(19) Europäisches Patentamt / European Patent Office / Office européen des brevets

(11) EP 1 346 220 B1

(12) **EUROPÄISCHE PATENTSCHRIFT**

(45) Veröffentlichungstag und Bekanntmachung des Hinweises auf die Patenterteilung:
**19.08.2009 Patentblatt 2009/34**

(21) Anmeldenummer: **01984880.3**

(22) Anmeldetag: **21.12.2001**

(51) Int Cl.:
***G01N 33/542*** *(2006.01)*

(86) Internationale Anmeldenummer:
**PCT/EP2001/015186**

(87) Internationale Veröffentlichungsnummer:
**WO 2002/054076 (11.07.2002 Gazette 2002/28)**

(54) **SENSOR ZUR LUMINESZENZ-OPTISCHEN BESTIMMUNG EINES ANALYTEN**

SENSOR FOR LUMINESCENCE OPTICAL DETERMINATION OF AN ANALYTE

CAPTEUR DESTINE A LA DETERMINATION OPTIQUE DE LUMINESCENCE D'UN ANALYTE

(84) Benannte Vertragsstaaten:
**AT BE CH CY DE DK ES FI FR GB GR IE IT LI LU MC NL PT SE TR**

(30) Priorität: **29.12.2000 AT 2161000**

(43) Veröffentlichungstag der Anmeldung:
**24.09.2003 Patentblatt 2003/39**

(73) Patentinhaber:
- **F. Hoffmann-La Roche AG**
  **4070 Basel (CH)**
  Benannte Vertragsstaaten:
  **AT BE CH CY DE DK ES FI FR GB GR IE IT LI LU MC NL PT TR**
- **Roche Diagnostics GmbH**
  **68305 Mannheim (DE)**
  Benannte Vertragsstaaten:
  **DE**

(72) Erfinder: **KLIMANT, Ingo**
**93098 Mintraching (DE)**

(74) Vertreter: **Schwarz, Albin**
**Kopecky & Schwarz**
**Patentanwälte**
**Wipplingerstraße 30**
**1010 Wien (AT)**

(56) Entgegenhaltungen:

| | |
|---|---|
| **WO-A-00/43780** | **WO-A-02/39083** |
| **WO-A-98/00714** | **US-A- 4 557 900** |
| **US-A- 4 868 103** | **US-A- 5 114 676** |
| **US-A- 5 194 393** | **US-A- 5 711 915** |
| **US-A- 5 942 189** | **US-B1- 6 348 322** |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein lumineszenz-optisches Verfahren zur qualitativen und quantitativen Bestimmung zumindest eines Analyten bzw. einer Komponente eines flüssigen Meßmediums, welche einen auf die zu bestimmende Komponente direkt oder indirekt durch Änderung seines Absorptionsspektrums reagierenden Chromophor (oder Luminophor) und einen auf die zu bestimmende Komponente nicht ansprechenden Luminophor aufweist, wobei das Emissionsspektrum des Luminophors zumindest teilweise mit dem Absorptionsspektrum des Chromophors überlappt und der strahlungslose Energietransfer zwischen Luminophor und Chromophor zumindest eine Lumineszenzeigenschaft des Luminophors meßbar ändert. Dieses Prinzip ist als sogenanntes FRET-Prinzip bekannt.

Die Erfindung betrifft weiters einen optisch-chemischen Sensor zur quantitativen Bestimmung zumindest eines Analyten bzw. einer Komponente eines gasförmigen oder flüssigen Meßmediums, welche einen auf die zu bestimmende Komponente direkt oder indirekt durch Änderung seines Absorptionsspektrums reagierenden Chromophor (oder Luminophor) und einen auf die zu bestimmende Komponente nicht ansprechenden Luminophor aufweist, wobei das Emissionsspektrum des Luminophors zumindest teilweise mit dem Absorptionsspektrum des Chromophors überlappt und der strahlungslose Energietransfer zwischen Luminophor und Chromophor zumindest eine Lumineszenzeigenschaft des Luminophors meßbar ändert.

Im folgenden werden unter Luminophore Farbstoffe verstanden, welche nach entsprechender Strahlungsanregung Phosphoreszenz- oder Fluoreszenzstrahlung emittieren. Das Absorptionsspektrum des Chromophors wird entweder direkt durch die zu messende Komponente oder indirekt durch ein chemisches Reaktionsprodukt der zu messenden Komponente beeinflußt. Unter "quantitative Bestimmungen einer chemischen Komponente" wird sowohl die Bestimmung der Konzentration, der Aktivität als auch des Gaspartialdruckes verstanden, wobei aus den Werten von zumindest einer Lumineszenzeigenschaft des Luminophors auf die Meßgröße geschlossen wird.

Ein Verfahren und ein Sensor, bei welchem pH- und kationensensitive Chromophore (Akzeptor) vorzugsweise kovalent an einen Luminophor (Donor) gebunden sind, sind aus der US 5,232,858 bekannt. Aus der Lumineszenzabklingzeit des Luminophors wird auf den pH-Wert bzw. auf die Konzentration des zu bestimmenden Kations im Meßmedium geschlossen.

Zum Stand der Technik ist ferner die US 5,648,269 zu nennen. Dieses Dokument schlägt die Verwendung der scheinbaren Lumineszenzabklingzeit des Luminophors zur Bestimmung der Meßgröße vor. Bei Luminophoren mit einer Abklingzeitkomponente ist die scheinbare Lumineszenzabklingzeit identisch der effektiven Abklingzeit. Bei Luminophoren mit mehreren Abklingzeitkomponenten ist die scheinbare Abklingzeit einfacher zu erheben, hat jedoch den Nachteil, daß - insbesondere bei nicht robusten Systemen - die Fehler größer werden.

Lumineszenzabklingzeiten können durch phasen-modulations bzw. zeitaufgelöste Lumineszenzmeßtechniken erhalten werden.

Ein ähnliches Verfahren ist aus der EP-A - 0 214 768 bekannt. Hier wird aus der gemessenen Lumineszenzintensität auf die Konzentration des zu bestimmenden Parameters im Meßmedium geschlossen.

Die strahlungslose Energietransferrate von Donor- zu Akzeptormolekülen hängt von der räumlichen Nähe der Moleküle beider Substanzen ab. Die Transferrate $k_T(r)$ ist extrem empfindlich vom räumlichen Abstand r zwischen Donor und Akzeptor und klingt mit der 6ten Potenz des Abstandes ab:

$$k_T(r) = \frac{1}{\tau_D}\left(\frac{R_o}{r}\right)^6,$$

wobei $\tau_D$ die Lumineszenzabklingzeit des Donors in Abwesenheit des Akzeptors und Ro die charakteristische Försterdistanz bedeuten. Letzteres ist jene Donor-Akzeptor Distanz, bei welcher eine 50%tige Effizienz des Energietransfers vorliegt. Je nach Donor-Akzeptor-Paar liegt Ro zwischen 2 und 9 Nanometer.

Infolge des strahlungslosen Energietransfers von Donor- zu Akzeptormolekülen ändern sich die makroskopisch bestimmbaren Werte der lumineszenzoptischen Parameter (Lumineszenzquantenausbeute, Lumineszenzintensität, Lumineszenzabklingzeit) des Luminophors besonders effizient, wenn eine substantielle Anzahl von Molekülen beider Substanzen in engen räumlichen Kontakt gebracht wird.

In der US 4,868,103 A, der US 5,194,393 A und der US 5,711,915 A werden Sensoren zur Durchführung immunoanalytischer Messungen beschrieben, in welchen lösliche, mit einem Donormolekül markierte Bindungskomponenten mit weiteren Bindungskomponenten reagieren, die auf einer mit Akzeptormolekülen versehenen Festphase immobilisiert sind.

Die US 5,942,189 A offenbart einen optischen FRET-Sensor zur Bestimmung von Analyten in einer Flüssigkeit, dessen Oberfläche mit einer Beschichtung zur Immobilisierung von Donor- und Akzeptormolekülen bedeckt ist.

Die US 5,114,676 A und die US 4,557,900 A beschreiben Sensorbeschichtungen mit zwei voneinander getrennten chemischen Phasen, wobei eine davon einen Fluorophor enthält und für das Probenmedium impermeabel ist.

Die WO 02/054476, ein Stand der Technik gemäß Art. 54(3) EPÜ, beschreibt eine auf dem FRET-Prinzip basierende Sensorvorrichtung, welche eine Vielzahl von Partikeln, z.B. aus Glas, umfasst, an deren Oberfläche fluoreszenzmarkierte Biomoleküle gebunden sind. Im Probenmedium werden andere fluoreszenzmarkierte Biomoleküle bereitgestellt, die mit den immobilisierten Biomolekülen Komplexe bilden und so zwischen den Fluoreszenzmarkern einen Energietransfer nach dem FRET-Prinzip ermöglichen.

Zur Herstellung des engen räumlichen Kontaktes wird in der US 5,232,858 die kovalente Verbindung von Donor- und Akzeptormolekülen vorgeschlagen. In der EP-A - 0 214 768 werden individuelle Donor- und Akzeptormoleküle kovalent an die Oberfläche eines gemeinsamen Substrates, z. B. Glas, gebunden.

Die in der US 5,232,858 beschriebene kovalente Verknüpfung von Donor- und Akzeptormolekülen hat den Vorteil, daß der mittlere räumliche Abstand von Donor/Akzeptor möglichst konstant gehalten werden kann. Nachteilig ist jedoch, daß der synthetische Aufwand zur Herstellung kovalenter Verbindungen vorteilhafter Luminophore mit geeigneten pH- oder ionensensitiven Chromophoren besonders hoch ist.

Die kovalente Anbindung von Donor- und Akzeptormolekülen an die Oberfläche eines gemeinsamen Substrates (EP-A - 0 214 768) ist ebenfalls sehr aufwendig und hat vor allem den Nachteil, daß Grenzflächeneffekte die Qualität der Meßergebnisse nachteilig beeinflussen.

In der US 5,942,189 und in der US 6,046,055 wird daher vorgeschlagen, daß der Luminophor und der Chromophor elektrisch unterschiedlich geladene ionische Substanzen sind, welche in Form von Ionenpaaren in einem für die zu bestimmende chemische Komponente permeablen Matrixmaterial vorliegen.

Für die breite kommerzielle Anwendung besonders wichtig ist die Verwendung langlebiger Luminophore (mit Lumineszenzabklingzeiten > 100ns, vorzugsweise > 1 $\mu$s), wie sie beispielsweise Metall-Ligand-Komplexe, bestimmte Porphyrine und Lanthanide aufweisen. Bei Vorliegen langlebiger Lumineszenz können die opto-elektronischen Anordnungen und Komponenten zur Bestimmung der Lumineszenzabklingzeit bzw. daraus ableitbare Größen (z.B. mittlere Lumineszenzabklingzeit, Phasenwinkel) mittels Phasen- oder zeitaufgelöster Methoden besonders kostengünstig bestimmt werden.

Der Erfinder der vorliegenden Anmeldung hat jedoch erkannt, daß die oben beschriebenen, vorbekannten Verfahren den gemeinsamen Nachteil haben, daß insbesondere die Lumineszenz langlebiger Luminophore durch eine Reihe von Komponenten des Meßmediums beeinflußt wird. Eine bekannte Eigenschaft derartiger Luminophore ist die besonders große Abhängigkeit der Lumineszenzeigenschaft vom 02 Gehalt der Probe. Konsequenterweise werden solche Luminophore daher häufig zur Bestimmung des 02-Gehaltes eingesetzt (EP-A - 0 907 074). Bei Verwendung dieser Luminophore als Donorfarbstoffe bei auf dem FRET-Prinzip basierenden Sensoren ist es daher erforderlich, den 02-Gehalt des Meßmediums genau zu kennen oder zu bestimmen und entsprechende Korrekturen durchzuführen. Beispiele für weitere, die Lumineszenzquantenausbeute beeinflussende Substanzen sind Amine und Wasser. Bei kontinuierlichen Messungen (Monitoring) können Lumineszenzfarbstoffe durch den entstehenden Singulett-02 ganz oder teilweise zerstört werden. Entsprechend wurden Additive vorgeschlagen, welche diesen Prozess eingrenzen sollen. Ein genereller Nachteil bekannter, vorteilhafter Lumineszenzfarbstoffe ist jedoch die Empfindlichkeit der Lumineszenzeigenschaften gegenüber geringfügigen Veränderungen der chemischphysikalischen Mikroumgebung, hervorgerufen durch beliebige Komponenten der Probe, insbesondere Wasser. Dies führt bei Probenmedien unbekannter bzw. variabler chemischer bzw. biochemischer Zusammensetzung zu einer signifikanten Einschränkung der Meßgenauigkeit. Beispielsweise werden in der medizinischen Diagnostik im Bereich der Bestimmung des Blut-pH Reproduzierbarkeiten von +/- 5 milli-pH erwartet.

Aufgabe der Erfindung ist es, auf dem FRET-Prinzip beruhende lumineszenzoptische Bestimmungsmethoden, basierend auf einem Luminophor (Donor) und einem Chromophor (Akzeptor, Indikator), welcher die zu bestimmende Substanz (Analyt) oder deren Reaktionsprodukte reversibel bindet, hinsichtlich der Störanfälligkeit durch Komponenten der zu messenden Probe zu verbessern. Ferner sollen besonders aufwendige chemische Syntheseschritte zur Realisierung der räumlichen Nähe einer substantiellen Anzahl von Akzeptor- und abgeschirmten Donormolekülen vermieden werden.

Diese Aufgabe wird erfindungsgemäß dadurch gelöst, daß der Luminophor (Donor) und der Chromophor (Akzeptor) jeweils in einem Matrixmaterial vorliegen und sich in unterschiedlichen chemischen Phasen befinden. Das Matrixmaterial der Donorphase soll für die Lumineszenzeigenschaften des Donors beinflussende Komponenten des Probenmediums essentiell impermeabel sein. Das Matrixmaterial der Akzeptorphase soll für den Analyten bzw. dessen Reaktionsprodukte permeabel sein.

Die Erfindung betrifft somit einen optisch-chemischen Sensor, der nach dem FRET-Prinzip arbeitet und einen auf einen in einem Probenmedium enthaltenen Analyt reagierenden Chromophor sowie einen Luminophor aufweist, und ist **dadurch gekennzeichnet, daß** der Luminophor und der Chromophor jeweils in einem Matrixmaterial vorliegen und sich in voneinander getrennten chemischen Phasen befinden, wobei die den Luminophor enthaltende Phase für das Probenmedium oder für die Lumineszenzeigenschaften des Luminophors beeinflussende Komponenten des Probenmediums impermeabel ist.

In einer Ausführungsvariante des erfindungsgemäßen Sensors ist vorgesehen, daß das Matrixmaterial der Akzeptor- und Donorphasen gemischt in bzw. in Form einer dünnen Schicht (Sensorschicht) vorliegt, welche auf einem transparenten Substrat oder an einem Lichtleiter befestigt sein kann. Zur Entkopplung der Sensorschicht von allfälligen störenden optischen Wechselwirkungen mit der zu analysierenden Substanz ist optional eine für den Analyten permeable optische Isolierschicht vorgesehen, welche sich zwischen der Sensorschicht und der zu analysierenden Substanz befindet.

In einer weiteren Ausführungsform (Abb. 5) besteht die Sensorschicht aus einem dünnen porösen Material. Die Poren dieses Materials enthalten das Matrixmaterial der Donorphase und das Matrixmaterial der Akzeptorphase.

In einer weiteren Ausführungsform (Abb. 6) wird die Sensorschicht aus einem dünnen Film gebildet, welche das Matrixmaterial der Akzeptorphase darstellt. Das Matrixmaterial der Donorphase liegt dabei homogen verteilt in Form von Partikeln im Matrixmaterial der Akzeptorphase vor. Die Akzeptorphase enthält die Akzeptormoleküle, homogen verteilt, und in einer ausreichenden Konzentration, sodaß sich hinreichend viele Akzeptormoleküle in der für den strahlungslosen Energietransfer erforderlichen räumlichen Nähe zu den in der Donorphase vorliegenden Donormolekülen befinden.

In einer weiteren Ausführungsform (Abb. 7) sind die Akzeptormoleküle an die Oberfläche der Partikel gebunden. Dabei kann der Chromophor adsoptiv oder elektrostatisch an der Oberfläche gebunden sein, oder, besonders bevorzugt, kovalent an funktionelle Gruppen gebunden sein. Die Bindung des Chromophores and die Partikeloberfläche hat vor allem den Vorteil, daß a) weniger Chromophor benötigt wird, b) optische Filtereffekt durch zu hohe Chromophorkonzentrationen der Akzeptorphase vermieden werden können, und c) das für die Rate des Energietransfers mitbestimmende Verhältnis von Donor- und Akzeptormolekülen bereits auf der Ebene der Herstellung der Partikel eingestellt werden kann und somit unabhängig von der Konzentration der Akzeptormoleküle in der Akzeptorphase ist.

Das erfindungsgemäße Verfahren zur qualitativen und/oder quantitativen Bestimmung zumindest eines Analyten bzw. einer Komponente eines gasförmigen oder flüssigen Meßmediums nach dem FRET-Prinzip ist gekennzeichnet durch die Verwendung eines erfindungsgemäßen Sensors.

Das erfindungsgemäße Verfahren dient bevorzugt zur Bestimmung des pH-Wertes einer Probe, zur Bestimmung der Konzentration bzw. der Aktivität von Ionen in einer Probe oder zur Bestimmung von in wässerigen Medien sauer oder alkalische reagierenden, unter Normalbedingungen gasförmig vorliegenden Komponenten.

Insbesondere dient das erfindungsgemäße Verfahren zur Bestimmung der Konzentration bzw. Aktivität von $Li^+$, $Na^+$, $K^+$, $Mg^{++}$, $Ca^{++}$ oder $Cl^-$.

Weiters bevorzugt dient das erfindungsgemäße Verfahren zur Bestimmung von $CO_2$ oder $NH_3$ in einem flüssigen Meßmedium.

Weitere Anwendungsgebiete des erfindungsgemäßen Verfahrens betreffen sogenannte Transducer. Hierbei reagiert der Chromophor nicht direkt mit dem Analyten, sondern indirekt. Beispiele hierfür sind sogenannten enzymatische Sensoren (bzw. zur Bestimmung von Harnstoff und Kreatinin). Hierbei reagieren ein oder mehrere Enzyme mit dem Analyten, wobei ein Produkt entsteht, welches direkt mit dem Chromophor reagiert.

Bevorzugt ist das Meßmedium eine Körperflüssigkeit, insbesondere Blut, Plasma oder Serum.

Besonders vorteilhafte, erfindungsgemäß eingesetzte Luminophore (Donor) sind solche, die sich durch eine hohe Lumineszenzquantenausbeute und eine lange Lumineszenzabklingzeit ( > 100 ns) auszeichnen. Bevorzugte Luminophore sind kationische metallorganische Komplexe des Palladiums, Rhodiums, Platins, Rutheniums, Osmiums, der seltenen Erden (insbesonders Europium und Lanthan). Der organische Teil dieser metallorganischen Komplexe kann z.B. aus Liganden aus der Gruppe der Porphyrine, Bipyridyle, Phenanthroline oder anderer heterozyklischer Verbindungen bestehen.

Bevorzugte pH- und kationensensitive Chromophore (Akzeptor) sind anionische Substanzen, deren Lichtabsorption sich durch direkte oder indirekte chemisch/physikalische Wechselwirkung mit der zu bestimmenden Komponente des Probenmediums verändert, und deren Absorptionsspektrum sich zumindest teilweise mit dem Emissionsspektrum des Luminophors überlappt.

I) Bestimmung des pH-Wertes einer Probe

Optische Sensoren zur Bestimmung des pH-Wertes enthalten gemäß Stand der Technik (M.J.P. Leiner and O.S. Wolfbeis "Fiber Optic pH Sensors" in O.S. Wolfbeis "Fiber Optic Chemical Sensors and Biosensors", CRC-Press, Boca Raton, 1991, Vol. I, Chapter 8) zumeist einen in einer ionenpermeablen, vorzugsweise hydrophilen Polymermatrix vorliegenden Absorptionsfarbstoff (Chromophor) oder Fluoreszenzfarbstoff. In Abhängigkeit des pH-Wertes (pH = -log (aH+)) des Probenmediums stellt sich ein thermodynamisches Gleichgewicht zwischen den protonierten und deprotonierten Formen des Chromophors bzw. Fluorophors ein. Aus dem mit optischen Methoden meßbaren Konzentrationsverhältnis beider Formen kann auf den pH-Wert des Probenmediums rückgeschlossen werden.

Reaktion:

$$AH \xleftrightarrow{K_d} A^- + H^+$$

Gleichgewicht

$$K_d = \frac{cA^- * cH^+}{cAH}$$

AH ist die protonierte und $A^-$ ist die deprotonierte Form des pH sensitiven Chromophors. $H^+$ steht für ein Proton. $K_d$ ist die Gleichgewichtskonstante. c steht für Konzentration.

In der eingangs erwähnten US 5,232,858 werden pH-sensitive Chromophore beschrieben, welche vorzugsweise kovalent an einen pH-insensitiven Luminophor (Donor) gebunden werden. Aus der Lumineszenzabklingzeit des Luminophors (L) wird auf den pH-Wert der Meßlösung geschlossen.

Zur erfindungsgemäßen lumineszenzoptischen pH-Bestimmung wird beispielsweise als Akzeptorfarbstoff ein pH-sensitiver Chromophor verwendet, welcher in der Akzeptorphase vorliegt bzw. zumindest in direktem Kontakt zur Akzeptorphase steht, wobei die Akzeptorphase auch das zu analysierende Medium (Meßmedium) sein kann.

Bei niedrigen pH-Werten (pH << pK des Chromophors) des Probenmediums liegt der Chromophor vollständig in der protonierten Form vor. Wegen der minimalen spektralen Überlappung der Absorptionsbande des deprotonierten Chromophors und der Emissionsbande des Luminophors erreicht die strahlungslose Energietransferrate vom Luminophor zum Chromophor ein Minimum. Entsprechend erreichen die Werte der mittleren Lumineszenzabklingzeit und der relativen Lumineszenzintensität des Luminophors ein Maximum.

Bei hohen pH-Werten (pH >> pKa des Chromophors) des Probenmediums liegt der Chromophor vollständig in der deprotonierten Form vor. Wegen der maximalen spektralen Überlappung der Absorptionsbande des deprotonierten Chromophors und der Emissionsbande des Luminophors erreicht die strahlungslose Energietransferrate vom Luminophor (Donor, Donorfarbstoff) zum Chromophor (Akzeptor, Akzeptorfarbstoff) ein Maximum. Entsprechend erreichen die Werte der mittleren Lumineszenzabklingzeit und der relativen Lumineszenzintensität des Luminophors ein Minimum.

Bei pH-Werten des Probenmediums im Bereich von +/- 1,5 pH-Einheiten vom pKd-Wert (pKd = -log Kd) des Chromophors kann aus der mittleren Lumineszenzabklingzeit oder der relativen Lumineszenzintensität des Luminophors mit ausreichender Genauigkeit auf den pH-Wert des Probenmediums geschlossen werden.

**II)** Bestimmung der Konzentration bzw. der Aktivität von Kationen und Anionen in einer Probe (Li+, Na+, K+, Mg++, Ca++, Cl-)

Bisher bekannt gewordene optische Sensoren bzw. optische Meßverfahren zur Bestimmung der Konzentration bzw. Aktivität von Kationen in einem Probenmedium beruhen auf unterschiedlichen Verfahren. So beschreibt die eingangs erwähnte erwähnten US 5,232,858 kationensensitive Chromophore, welche vorzugsweise kovalent an einen kationeninsensitiven Luminophor gebunden werden.

Bei sehr hohen Kationenkonzentrationen ($cY^{+p}$ >> Kd des Chromophors) des Probenmediums liegt der Chromophor vollständig in der komplexierten Form vor. (Y ist das zu bestimmende Kation, +p ist seine Ladungszahl). Bei sehr niedrigen Kationenkonzentrationen ($cY^{+p}$ << Kd des Chromophors) des Probenmediums liegt der Chromophor in freier, unkomplexierten Form vor.

Liegt die logarithmische Konzentration log($cY^{+p}$) des zu bestimmenden Kations des Probenmediums im Bereich von -log(Kd) +/- 1.5, so kann mit ausreichender Genauigkeit aus der mittleren Lumineszenzabklingzeit bzw. der relativen Lumineszenzintensität des Luminophors auf die Konzentration des zu bestimmenden Kations im Probenmedium geschlossen werden.

Weitere optische Meßverfahren bzw. Sensoren zur Bestimmung der Konzentration bzw. Aktivität von Kationen, sind z. B. aus der US-A 4,645,744, EP-A - 0 358 991 und der EP-A - 0 461 392 bekannt geworden, wobei ein pH-sensitiver Chromophor bzw. ein pH-sensitiver Luminophor und ein ladungsneutraler Ionophor in einer im wesentlichen hydrophoben Polymermatrix vorliegen. Das geoffenbarte Meßverfahren basiert darauf, daß Kationen ($Y^{+p}$) mit dem Probenmedium (beispielsweise $K^+$ gegen $H^+$ oder $Ca^{++}$ gegen $2H^+$) ausgetauscht werden. Infolgedessen sind die Meßergebnisse abhängig vom pH-Wert des Probenmediums. Derartige Meßverfahren sind für jene Meßsituationen geeignet, wo der pH-Wert des Probenmediums bekannt ist oder mittels einer pH-Pufferschicht auf einen bekannten Wert eingestellt werden kann.

In einer erfindungsgemäßen Weiterbildung dieser Methoden werden zur Bestimmung der Kationenkonzentration ein pH-sensitiver Chromophor, welcher sich in der Akzeptorphase befindet oder diese zumindest kontaktiert, und ein pH-insensitiver Luminophor, welcher sich in der Donorphase befindet, sowie ein für das zu bestimmende Kation selektiver ladungsneutraler Ionophor (I), welcher sich in der Akzeptorphase befindet, vorgesehen.

Aus der EP-A - 0 358 991 und aus Anal. Chim. Acta 255 (1991), p. 35-44 sind optische Sensoren zur Bestimmung von Anionen, beispielsweise $Cl^-$ bekannt, wobei das zu bestimmende Anion zusammen mit einem Kation aus dem Meßmedium co-extrahiert wird (beispielsweise $Cl^-$ und $H^+$). Hier liegen ein lipophiler pH-sensitiver Chromophor (Fluoresceinderivat) und eine optisch inaktive kationische Substanz ($Q^+$) in einer im wesentlichen hydrophoben Polymermatrix vor.

In Abhängigkeit der $H^+$- und der $Cl^-$-Konzentration des Meßmediums liegt der in der Polymermatrix befindliche pH-sensitive Chromophor in der protonierten und/oder deprotonierten Form vor. Mit zunehmendem Deprotonierungsgrad steigt die Absorption der deprotonierten Form. Der Deprotonierungsgrad (und infolge dessen die Absorption) ist abhängig vom pH-Wert und der Konzentration des zu bestimmenden Anions. Der pH-Wert des Meßmediums muß bekannt sein bzw. auf einen bekannten Wert eingestellt werden, um auf die Konzentration des zu bestimmenden Anions schließen zu können.

In einer erfindungsgemäßen Weiterbildung der geoffenbarten Methode werden beispielsweise zur Bestimmung der Chloridkonzentration eines Meßmediums ein pH-sensitiver Chromophor, welcher sich in einer essentiell hydrophoben, von Chloridionen des Meßmediums mittels Coextraktion permeablen Akzeptorphase befindet bzw. diese kontaktiert (wobei die Akzeptorphase NICHT das Meßmedium sein kann), ein Luminophor, welcher sich in einer Donorphase befindet, sowie eine lipophile kationischen Substanz (Ionophor) vorgesehen. Die lipophile Substanz (Q+) kann z. B. eine quaternierte Ammoniumverbindung sein.

Erfindungsgemäß eingesetzte pH-sensitive Chromophore sind unten in Tab. 1 beispielhaft angeführt. Bei niedrigen pH-Werten und hohen Chloridkonzentrationen des Meßmediums liegt der pH-sensitive Chromophor vorzugsweise in der protonierten Form vor und die optisch inaktive kationische Substanz bildet in der Matrix ein Gegenion zu $Cl^-$. Die Absorption der deprotonierten Chromophorform erreicht ein Minimum. Die Werte der mittleren Lumineszenzabklingzeit und der relativen Lumineszenzintensität des Luminophors erreichen ein Maximum.

Bei hohen pH-Werten und niedrigen Chloridkonzentrationen des Meßmediums liegt der pH-sensitive Chromophor vorzugsweise in der deprotonierten Form vor, wobei die optisch inaktive kationische Substanz die durch die Dissoziation des Protons entstandene negative Ladung kompensiert. Die Absorption der deprotonierten Chromophorform erreicht ein Maximum. Die Werte der mittleren Lumineszenzabklingzeit und der relativen Lumineszenzintensität des Luminophors erreichen ein Minimum.

Ist der pH-Wert des Meßmediums bekannt, kann aus den Werten der mittleren Lumineszenzabklingzeit bzw. der relativen Lumineszenzintensität des Luminophors auf die Chloridkonzentration des Meßmediums geschlossen werden.

**III**) Bestimmung von in wässriger Umgebung schwach sauer oder basisch reagierender, unter normalen Bedingungen gasförmig vorliegender Komponenten flüssiger oder gasförmiger Meßmedien:

Bestimmung von $CO_2$

Optische Sensoren zur Bestimmung des $CO_2$ -Partialdruckes eines flüssigen oder gasförmigen Meßmediums bestehen zumeist aus einem Reaktionsraum, welcher von einem ionenimpermeablen, gaspermeablen Material vom Meßgut getrennt wird. Der Reaktionsraum ist häufig identisch mit dem indikatortragenden Material eines optischen pH-Sensors. Zudem befinden sich üblicherweise im Reaktionsraum eine oder mehrere pH-Puffersubstanzen, beispielsweise Carbonate, Phosphate, und/oder in wässrigen Medien sauer oder basisch reagierende organische Verbindungen. Die p $CO_2$ -Bestimmung des Meßmediums kann somit auf eine optische pH-Bestimmung zurückgeführt werden.

In einer in der EP-A - 0 105 870 beschriebenen Ausführungsvariante dieses Meßprinzips liegt der Reaktionsraum in Form von "Tröpfchen" in einem ionenimpermeablen, gaspermeablen Polymermaterial vor. In einer in der US 5,496,521 beschriebenen Ausführungsvariante dieses Meßprinzips liegt der Reaktionsraum in Form einer hydrophilen Schicht, bedeckt von einem ionenimpermeablen, gaspermeablen Polymermaterial vor.

Reaktion:

$$CO_2 + H_2O \longleftrightarrow H_2CO_3$$

$$H_2CO_3 \longleftrightarrow HCO_3^- + H^+$$

$$AH \longleftrightarrow A^- + \mathrm{H}^+$$

AH ist die protonierte und $A^-$ ist die deprotonierte Form des Chromophors. $H^+$ steht für ein Proton.

In einer erfindungsgemäßen Weiterbildung der lumineszenzoptischen $CO_2$ -Bestimmung liegen in einem hydrophilen, vom Meßmedium durch ein ionenimpermeables, gaspermeables Material getrennten Reaktionsraum ein erfindungsgemäß von chemischen Parametern abgeschirmter Donor und ein pH-sensitiver Akzeptorfarbstoff vor.

Die $CO_2$-Bestimmung mit erfindungsgemäßen Sensoren bestehend aus einem, einen wässrigen pH-Puffer enthaltenden Reaktionsraum und einem, den Reaktionsraum vom Meßmedium trennenden ionenimpermeablen gaspermeablen Material, wird auf eine Bestimmung des pH-Wertes im Reaktionsraum des Sensors zurückgeführt. Hohe $CO_2$-Werte des Meßmediums entsprechen niederen pH-Werten des Reaktionsraumes und niedrige $CO_2$-Werte des Meßmediums entsprechen hohen pH-Werten des Reaktionsraumes.

Ein alternatives Verfahren zur optischen $CO_2$ -Bestimmung, welche mit einem einzigen Reaktionsraum ohne Zuhilfenahme wässriger pH-Puffersubstanzen auskommt, wurden von Mills et al. in Anal. Chem. 64, 1992, 1383-1389 beschrieben. Hierbei liegt die deprotonierte Form eines pH-sensitiven Chromophors mit einer optisch inaktiven kationischen Substanz ($Q^+$) in einem Reaktionsraum bestehend aus einem essentiell ionenimpermeablen, gaspermeablen Polymermaterial vor. In das Polymermaterial diffundierendes $CO_2$ des Meßmediums wird hydratisiert und reagiert in einer chemischen Gleichgewichtsreaktion mit der deprotonierten Chromophorform und der optisch-inaktiven Substanz. Aus der Lichtabsorption der deprotonierten Chromophorform wird auf den $CO_2$ -Partialdruck des Meßmediums geschlossen.

Reaktion:

$$CO_2 + H_2O \longleftrightarrow H_2CO_3$$

$$\left(A^- Q^+\right) + H_2CO_3 \longleftrightarrow \left(Q^+ HCO_3^-\right) + AH$$

In einer erfindungsgemäßen Variante der lumineszenzoptischen $CO_2$-Bestimmung werden ein essentiell lipophiler, pH-sensitiver Chromophor, welcher sich in einer, im Fall wäßriger Meßmedien für ionische Substanzen essentiell impermeablen Akzeptorphase befindet bzw. diese kontaktiert, ein Luminophor, welcher sich in einer Donorphase befindet, sowie essentiell lipophile kationische Substanz welche sich in der Akzeptorphase befindet, vorgesehen.

Reaktion:

$$CO_2 + H_2O \longleftrightarrow H_2CO_3$$

$$\{A^-Q^+\} + nH_2CO_3 \longleftrightarrow n(Q^+HCO_3^-) + AH$$

Die $CO_2$-Bestimmung mit einem Sensor, bestehend aus einem Reaktionsraum ohne wässrige Puffersubstanzen, wird erfindungsgemäß auf die Bestimmung des Verhältnisses der protonierten und deprotonierten Formen eines pH-sensitiven Chromophors zurückgeführt.

Bei sehr niedrigen $CO_2$-Werten des Meßmediums liegt der Chromophor vorzugsweise in der deprotonierten Form vor und bildet in der Matrix eine ionale Verbindung mit der optisch inaktiven kationischen Substanz. Die Absorption der deprotonierten Chromophorform erreicht ein Maximum. Die Werte der mittleren Lumineszenzabklingzeit und die relativen Lumineszenzintensität des Luminophors erreichen ein Minimum.

Bei hohen $CO_2$-Werten des Meßmediums liegt der Chromophor in der protonierten Form vor. Die optisch inaktive kationische Substanz bildet eine ionale Verbindung mit Hydrogen-carbonat. Die Absorption der deprotonierten Chromophorform erreicht ein Minimum. Die Werte der mittleren Lumineszenzabklingzeit und die relativen Lumineszenzintensität des Luminophors erreichen ein Maximum.

Bestimmung von $NH_3$

Die Bestimmung von $NH_3$, als Beispiel für eine in wässriger Umgebung basisch reagierende Komponente kann ähnlich der Bestimmung von $CO_2$ nach Mills. (T.Werner et al., Analyst 120, 1995, 1627-1631) erfolgen. Es wird keine optisch inaktive kationische Substanz benötigt.

Reaktion:

$$AH + NH_3 \longleftrightarrow (A^-NH_4^+)$$

Die erfindungsgemäße Bestimmung von $NH_3$ erfolgt in Analogie zur erfindungsgemäßen $CO_2$-Bestimmung:

Erfindungsgemäß anwendbare pH-sensitive Chromophore:

Tabelle 1: pH-sensitive Chromophore

| Chromophor | Absorptionswellenlänge [nm] protoniert/deprotoniert | pKa |
|---|---|---|
| Triphenylmethanfarbstoffe: | | |
| Bromphenolblau | 430 / 617 | 3.8 |
| Bromthymolblau | 430-435 / 615-618 | 6.7 |
| Dibromoxylenolblau | 420/614 | 7.6 |
| Azofarbstoffe: | | |
| Calmagit | 530/605 | 8.0 |
| Nitrazingelb | 460/590 | 6.5 |
| Sonstige: | | |
| o-Chlorophenol-indophenol | 555/625 | 7.1 |
| Naphthol-phthalein | 428/661 | 6.7,7.9 |

Weiters finden pH-sensitive Triphenylmethanfarbstoffe der allgemeinen Form

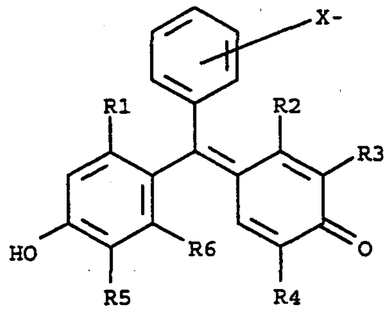

Verwendung. R1-6 können unabhängig voneinander H, Halogenatome, Nitrogruppen und Alkylgruppen sein. $X^-$ steht für eine optionelle Gruppe zur kovalenten Immobilisierung des Chromophors. Geeignete Gruppen sind beispielsweise -(CH2)n-$SO3^-$ oder -(CH2)n-$COO^-$, - (CH2)n-NH2 (n=0-18)
pH sensitive Azofarbstoffe der allgemeinen Form,

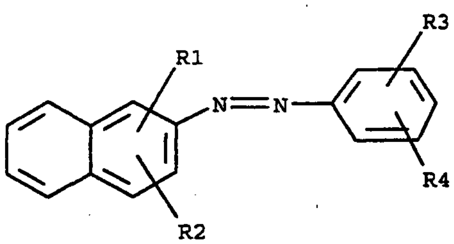

wobei R1-R4 unabhängig voneinander Substituenten, beispielsweise Halogenatome, Nitrogruppen bzw. Alkylgruppen und für die kovalente Immobilisierung geeignete Gruppen sind, - aber mindestens eine -OH Gruppe vertreten sein muß.

Erfindungsgemäß anwendbare kationensensitive Chromophore (Chromoionophore): Erfindungsgemäß anwendbare, kationensensitive Chromoionophore zur Bestimmung von Lithium-, Kalium-, Natrium-, Magnesium- und Calcium-Ionen sind beispielsweise anionische Azofarbstoffe, Stilbenfarbstoffe und Merocyanine welche zumindest eine ionenselektive Gruppe (Ionophore Gruppe) enthalten und deren längstwellige Absorptionsbande sich zumindest teilweise mit der Emissionsbande des Luminophors überlappt, wobei die Wechselwirkung mit dem zu bestimmenden Kationen zu einer spektralen Verschiebung der längstwelligen Absorptionsbande führt.

Neutrale Ionophore sind unten in der Tabelle 2 angegeben.

Tabelle 2: Neutrale Ionophore (geeignet für Ion-Exchange) Sensoren

| Ionophor | Ion |
|---|---|
| PTM14C4 (14-Krone-4) **) | Li+ |
| Natrium-Ionophor I bis II *) | Na+ |
| Valinomycin | K+ |
| Magnesium Ionophor ETH 3832 *) | Mg++ |
| Calcium Ionophor I-IV *) | Ca++ |

*) Ionophores for Ion-Selective Electrodes and Optodes. Fluka Chemie AG, CH-9470 Buchs, Switzerland.
**) K. Wanabe et al. Anal. Chem. 65, 1993.

Tabelle 3: Luminophore (Donorfarbstoffe)

| Luminophor (L) | Abkürzung | Absorptions-maximum (nm) | Lumineszenz-maximum (nm) |
|---|---|---|---|
| (Ru(II)-tris-2,2'-bibyridyl)$^{2+}$ | $Ru(bpy)_3^{2+}$ | 452 | 628 |

(fortgesetzt)

| Luminophor (L) | Abkürzung | Absorptions-maximum (nm) | Lumineszenz-maximum (nm) |
|---|---|---|---|
| (Ru(II)-tris-2,2' 4,4-diphenyl bipyridyl)$^{2+}$ | Ru(dph-bpy)$_3^{2+}$ | 474 | 632 |
| (Ru(II)-tris-1,10-phenanthrolin)$^{2+}$ | Ru(phen)$_3^{2+}$ | 447 | 604 |
| (Os(II)-bis-terpyridin)$^{2+}$ | | 510 | 729 |
| (Os(II)-tris-1,10-phenanthrolin)$^{2+}$ | | 650 | 690 |

Als Zentralatome finden auch Ir, Rh, Pd, Pt oder Re Verwendung.

Essentiell hydrophobe, ionenimpermeable Akzeptorphasen:

Zur Herstellung erfindungsgemäßer Sensoren, welche das zu bestimmende Kation (beispielsweise K+) mit einem Proton des Meßmediums austauschen bzw. das zu messende Anion (beispielsweise Cl-) mit einem Proton des Meßmediums co-extrahieren, sind für ionale Substanzen des Meßmediums substantiell impermeable Polymermaterialien geeignet.

Diese Materialien sind auch geeignet zur Herstellung erfindungsgemäßer Sensoren zur Bestimmung von Gasen bzw. gasförmiger Komponenten flüssiger Meßmedien.

Bevorzugt werden alle essentiell hydrophoben, in organischen Lösungsmitteln lösliche Polymere wie beispielsweise Polyvinylchlorid, Polystyrole, Polycarbonate, Polyethylene, Polyurethane, Silikone, Copolymere aus Polyvinylalkohol und Polyvinylacetat und Copolymere aus Polyvinylchlorid, Polyvinylalkohol und/oder Polyvinylacetat.

Diesen Materialien können bis zu 80 Gew. % Weichmacher wie beispielsweise Dioctylsebacat, Tris-(2-ethylhexyl)-Phosphat, 2-Nitrophenyl-octyl-ether, 2-Nitrophenyl-butylether zugesetzt werden.

Essentiell hydrophile, ionenpermeable Akzeptorphasen:

Zur Herstellung erfindungsgemäßer Sensoren mit pH- und ionensensitiven Chromophoren werden hydrophile, ionenpermeable Polymere bevorzugt.
Beispielhaft dafür seien Zellulose, Polyurethane mit hydrophilen Gruppen, Polyhydroxyethylmethacrylate, vernetzte Polyvinylalkohole, und/oder Polyacrylamide genannt.

Geeignete Materialien für Donorphasen sind z.B. harte, nicht weichgemachte Polymere wie Polyacrylnitril und Derivate, PVC und Polyvinylidenchlorid.

Die Erfindung wird anhand der Abb. 1 bis 7 näher erläutert, wobei

Abb. 1 eine Kalibrationskurve eines pH-Reagens;
Abb. 2 eine Kalibrationskurve eines erfindungsgemäßen pH-Sensors;
Abb. 3 eine Kalibrationskurve eines $CO_2$-Sensors gemäß dem Stand der Technik;
Abb. 4 eine Kalibrationskurve eines erfindungsgemäßen $CO_2$-Sensors und die
Abb. 5 bis 7 jeweils eine Ausführungsform eines erfindungsgemäßen Sensors in schematischer Darstellung, wie auf Seite 5 beschrieben, zeigen.

Bestimmung der Kalibrierwerte

Die Bestimmung der Kalibrierkurven (Abbildungen 1-4) und 02-Abhängigkeiten erfolgte durch die Bestimmung der Phasenverschiebung der Lumineszenz bezüglich des sinusförmig modulierten Anregungslichtes. Da ein langlebiger Luminophor verwendet wird, genügt eine einfache Meßanordnung mit einer Modulationsfrequenz von 45 kHz.

Zur Messung wurden Sensorscheiben aus den Sensorfolien herausgestanzt, diese am Ende eines 2-armigen Lichtleiterbündels befestigt und mit den jeweiligen Meßmedien in Kontakt gebracht bzw. das Ende des Lichtleiterbündels direkt in das, die Probe enthaltende Messmedium getaucht. Als Anregungslichtquelle wurde eine blaue LED (470 nm) verwendet, die mit einer auf 45 kHz sinusoidal modulierten Spannung der Amplitude 5 V versorgt wurde. Als Filter für das Anregungslicht wurden blaue Folienfilter verwendet. Das Anregungslicht wurde mittels Lichtleiter zur Sensorfolie bzw. Messflüssigkeit geführt. Das emittierte Lumineszenlicht wurde mittels Lichtleiterbündel zu einem Filter, eine Kombination aus einem OG 570 Glas Filter (Schott) und einem roten Folienfilter, geführt und weiters auf einen Detektor

(Photomultiplier, Type Hamamatsu H5702) geleitet. Die sinusförmig modulierte Versorgungsspannung der LED und das Signal des Detektors wurde mittels eines Lock-In Verstärkers ausgewertet. Als Meßsignal wurde die Phasenverschiebung $\Phi$ erhalten.Die Abklingzeit $\tau$ wurde aus der gemessenen Phasenverschiebung $\Phi$ und der Modulationsfrequenz f = 45 kHz mit folgender Formel berechnet : $\tau = \tan(\Phi)/ (2 \pi f)$.

Als Meß- bzw. Kalibriermedien für pH Probe (Abb 1) bzw. pH Sensoren (Abb 2) wurden Phosphatpuffer verwendet, welche mit NaOH bzw. HCl auf die gewünschten pH-Werte eingestellt wurden. Die Puffer wurden durch Tonometrie mit Luft auf den Luftsauerstoffwert eingestellt bzw. durch Zugabe von $Na_2SO_3$ sauerstoffrei gemacht. Als Kalibriermedien für $CO_2$ Sensoren (Abb. 3 und 4) wurden Mischgase mit unterschiedlicher Zusammensetzung an $N_2$, $O_2$ und $CO_2$ verwendet.

Abb. 1 zeigt die Kalibrationskurve eines auf dem FRET-Prinzip beruhenden optischen pH-Reagens (hergestellt nach 2.3) mit pH-sensitivem Akzeptorfarbstoff, wobei der Donorfarbstoff "geschützt" in einer erfindungsgemäßen Donorphase vorliegt. Die beiden Kurven zeigen den Phasenwinkel (Ordinate) des Lumineszenzlichts der in Kalibrationsflüssigkeiten unterschiedlicher pH-Werte (Abszisse) dispergierten pH Probe. Die mit "O" bezeichnete Kurve wurde mit Luft (21,95% $O_2$) gesättigt aufgenommen. Die mit "N" gekennzeichnete Kurve wurde mit $N_2$ gesättigt aufgenommen.

Abb. 2 zeigt die Kalibrationskurve eines auf dem FRET-Prinzip beruhenden optischen pH Sensors (hergestellt nach 2.4), wobei der Donorfarbstoff "geschützt" in einer erfindungsgemäßen Donorphase vorliegt. Die beiden Kurven "N" und "O" beziehen sich auf die gleichen Proben wie für Abb. 1 erklärt und veranschaulichen den Phasenwinkel (Ordinate) mit Kalibrationsflüssigkeiten unterschiedlicher pH-Werte (Abszisse).

Abb. 3 zeigt die Kalibrationskurve eines auf dem FRET-Prinzip beruhenden optischen $CO_2$ Sensors (hergestellt nach 3.1), wobei der Donorfarbstoff "ungeschützt" in der Akzeptorphase vorliegt. Die beiden Kurven veranschaulichen die Lumineszenzabklingzeiten (Ordinate) mit Kalibrationsgasen unterschiedlichen Partialdrucks an $CO_2$ (Abszisse). Die Kurve "OF" bedeutet sauerstofffrei. Die Kurve "AC" bedeutet "Luft-Zustand" mit 21,95 % $O_2$.

Abb. 4 zeigt die Kalibrationskurve eines auf dem FRET-Prinzip beruhenden optischen $CO_2$ Sensors (hergestellt nach 3.2), wobei der Donorfarbstoff "geschützt" in einer erfindungsgemäßen Donorphase vorliegt. Die beiden Kurven (Bedeutungen von "OF" und "AC" wie bei Abb. 3) veranschaulichen die Lumineszenzabklingzeiten (Ordinate) mit Kalibrationsgasen unterschiedlichen Partialdrucks an $CO_2$ (Abszisse).

Abb. 5 zeigt eine bevorzugte Ausführungsform des erfindungsgemäßen Sensors. Dabei bedeuten:

1 Optisch transparenter Träger
2 Optische Isolierschicht (optional)
3 In den Sensor einfallendes und. aus diesem austretendes Licht.
51 Poröses Material
52 Donorphase mit Donorfarbstoff (kleine Quadrate)
53 Akzeptorphase mit Akzeptorfarbstoff (kleine Kreise)

Abb. 6 zeigt eine weitere bevorzugte Ausführungsform des erfindungsgemäßen Sensors, wobei die Bezugsziffern 1, 2 und 3 die gleiche Bedeutung haben wie in Abb. 5. Weiters bedeuten:

61 Donorphase mit Donorfarbstoff (größere Kreise)
62 Akzeptorphase mit Akzeptorfarbstoff (kleinere Kreise)

Abb.7 zeigt eine weitere bevorzugte Ausführungsform des erfindungsgemäßen Sensor, wobei die Bezugsziffern 1, 2 und 3 die gleiche Bedeutung haben wie in Abb. 5. Weiters bedeuten:

71 Donorphase mit Donorfarbstoff (größere Kreise) und gebundenem Akzeptorfarbstoff (kleinere Kreise)
72 Akzeptorphase

BEISPIELE

$Ru(diphphen)_3TMS_2$
[=Ru(II)-tris-(4,7-diphenyl-1,10-phenanthroline)(3-trimethylsilyl)-1-propane sulphonate] (J.Klimant und O.S. Wolfbeis, Anal. Chem. 67 (1995) 3160).

Beispiel 1

Mit diesem Beispiel wird dokumentiert, daß ein Donorfarbstoff, welcher erfindungsgemäß in einer Donorphase eingebaut ist, weniger 02 Empfindlichkeit aufweist als derselbe Farbstoff, eingebaut in der Akzeptorphase.

Allgemeine Beschreibung der Herstellung von Nanopartikeln mit eingebautem Donorfarbstoff.

1.1 Einbetten eines Donorfarbstoffs in eine Akzeptorphase

1 mg des Donorfarbstoffs Ru(diphphen)$_3$TMS$_2$ und 100 mg des hydrophilen Polymers D4 (Polyurethan mit hydrophilen Sequenzen; Tyndale Plains Hunter LTD, Ringoes, NJ 08551, USA) wurden in Ethanol:Wasser (90:10 w/w) gelöst. Die Lösung wurde durch Rakeln auf eine Polyesterfolie (Mylar, Dupont) aufgezogen. Nach Abdunsten des Lösungsmittels entstand ein Film mit einer Schichtdicke von ca. 20 $\mu$m.

Messanordnung (45 kHz, blaue LED, OG 570)

| | | | | |
|---|---|---|---|---|
| Trocken: | Luft | 55.4° | N2: | 58,3° |
| Wasser: | Luft gesättigt: | 53.5° | N2 gesättigt: | 58.4° |

Das Meßergebnis zeigt, daß der "ungeschützt" in einer Akzeptorphase vorliegende Donorfarbstoff Ru(diphphen)$_3$TMS$_2$, in Kontakt mit 02-freien (N2-Sättigung) bzw. 21.95% 02 (Luft) gesättigten gasförmigen und wässrigen Medien, eine 02-Empfindlichkeit von 2.9° (trockene Akzeptorphase) bzw. 4.9° (nasse Akzeptorphase) aufweist.

1.2 Herstellung der Nanopartikel mit Donorfarbstoff

400 mg Polyacrylnitril (Polyscience) und 8 mg Ru(diphphen)$_3$TMS$_2$ wurden in 80 ml DMF (Merck) gelöst. Nach langsamem Zutropfen von 500 ml Wasser wurde die entstandene Suspension mit NaCl versetzt und zentrifugiert. Das Zentrifugat wurde mehrmals mit Wasser und anschließend mit Ethanol gewaschen.

1.3 Einbetten der Donorfarbstofftragenden Nanopartikel in eine schichtförmige Akzeptorphase.

Die ethanolische Suspension (aus 1.2) wurde mit einer Lösung von 400 mg des hydrophilen Polymers D4 (Tyndale Plains Hunter LTD, Ringoes, NJ 08551, USA) in 5 ml Ethanol:Wasser (90:10, w:w) versetzt. Die Suspension wurde durch Rakeln auf eine Polyesterfolie (Mylar, Dupont) aufgezogen. Nach Abdunsten des Lösungsmittels entstand ein Film einer Schichtdicke von ca. 20 $\mu$m.

Messanordnung (45 kHz, blaue LED, OG 570)

| | | | | |
|---|---|---|---|---|
| Trocken: | Luft | 57,4°; | N2: | 58,2° |
| Wasser: | Luftgesättigt: | 56.8° | O2-frei ($SO_3^{2-}$): | 58.6° |

Das Meßergebnis zeigt, daß der in ein erfindungsgemäßes Nanopartikel eingebaute, und somit "geschützt" in der Donorphase vorliegende Donorfarbstoff Ru(diphphen)$_3$TMS$_2$, in Kontakt mit 02-freien bzw. 21.95% 02 gesättigten, gasförmigen bzw. wässrigen Medien, eine 02-Empfindlichkeit von 0.8° (trockene Akzeptorphase) bzw. 1.6° (nasse Akzeptorphase) aufweist.

Durch Vergleich dieser Werte mit den Werten aus 1.1 wird somit ersichtlich, daß ein und derselbe in einer erfindungsgemäßen Donorphase vorliegende Donorfarbstoff weniger 02 Empfindlichkeit aufweist als der nach dem Stand der Technik direkt in der Akzeptorphase vorliegende Donorfarbstoff.

Beispiel 2 (pH-Sensor)

Allgemeine Beschreibung der Herstellung pH-funktioneller Nanopartikel und einer pH-sensitiven Schicht.

In einem ersten Schritt wird ein OH-funktionelles Co-Polymer aus Acrylonitril und einem OH funktionellen Methacrylat hergestellt. In einem zweiten Schritt werden aus diesem Polymer Nanopartikel mit eingebautem Donorfarbstoff hergestellt. In einem dritten Schritt wird ein pH-sensitiver Akzeptorfarstoff kovalent an die funktionellen Gruppen des Co-Polymers gebunden. Der Akzeptorfarbstoff ist dabei überwiegend in den "weichen" hydrophilen Bereichen (Akzeptorphase) der Partikel lokalisiert, und somit für Ionen zugänglich. Der Donorfarbstoff ist überwiegend in den "harten" Bereichen (Donorphase) gelöst und damit für störende Substanzen erschwert zugänglich. In einem vierten Schritt werden die Partikel in einer Lösung eines hydrophilen Polymers suspendiert und die Lösung auf ein geeignetes Trägermaterial

aufgebracht. Nach dem Verdunsten des Lösungsmittels entsteht ein pH-sensitiver Sensorfilm.

2.1 Herstellung des Co-Polymers

230 g entionisiertes $H_2O$ wurden durch 2 h Spülen mit Stickstoff sauerstoffrei gemacht. Unter Rühren und Stickstoffatmosphäre wurden 4g SDS (Natriumdodecylsulfat p. A., Merck) gelöst. Zu dieser Lösung wurden 20 ml Acroylnitril (Fluka) und 1 ml Polyethylenglycolmonometacrylat (Polyscience, n=200, Bestell Nr. 16712) gegeben. Die Mischung wurde auf 50° gebracht und mit 4 ml 1 N HCl (Merck) versetzt. Die Polymerisierung wurde durch Zugabe von 400 mg Ammoniumperoxodisulfat (Merck) gestartet und 12 h bei 50° durchgeführt. Das Polymer wurde abgesaugt und mehrmals mit Wasser und Ethanol gewaschen. Dieses Polymer wird im folgenden als PAN-PEG bezeichnet.

2.2 Herstellung der Nanopartikel mit Donorfarbstoff

400 mg PAN-PEG und 20 mg des Donorfarbstoffs $Ru(diphphen)_3TMS_2$ wurden in 80 ml DMF (Merck) gelöst. Nach langsamen Zutropfen von 500 ml Wasser wurde die Suspension mit NaCl versetzt und zentrifugiert. Das Zentrifugat wurde mehrmals mit Wasser gewaschen.

2.3 Binden des pH-sensitiven Akzeptorfarbstoffes Chromophors an die Nanopartikel

25 mg des pH-sensitiven Akzeptorfarbstoffs N9 (Merck) wurden mit 8 Tr. $H_2SO_4$ konz. (Merck) verrieben und 30 min im Wasserstrahlvakkum aktiviert. Der Farbstoff wurde in 100 ml entionisiertem Wasser aufgenommen und mit NaOH auf pH 7 gebracht. Zu dieser Mischung wurde das oben beschriebene Zentrifugat gegeben, nach 5 min 4.2 g $NaCO_3$, nach 5 min 2 ml 5 M NaOH zugegeben. Nach weiteren 20 min wurde mit HCL konz. auf pH 3 angesäuert. Die Partikel wurden durch Zentrifugation abgetrennt und mit basischen Puffer, Wasser und Ethanol gewaschen.

Die so hergestellten Partikel wurden in ein wässriges Meßmedium suspendiert und die Messungen bei verschiedenen pH-Werten unter Luft- und N2-Sättigung vorgenommen. Das Ergebnis ist in der Abbildung 1 dargestellt und zeigt, daß die pH-sensitiven Partikel auch als Reagens (=Sonde bzw. engl. "Probe") zwecks pH-Bestimmung dem Meßmedium zugesetzt werden können.

| | | | | | |
|---|---|---|---|---|---|
| Meßmedium: | pH 7.3 | Luftgesättigt: | 50.0° | O2-frei (N2 gesättigt): | 51.3° |

2.4 Herstellung der pH sensitiven Schicht (mit Partikeln nach 1.3) eines optischen pH Sensors Die ethanolische Suspension (2.3) wurde mit einer Lösung von 400 mg des hydrophilen Polymers D4 (Tyndale Plains Hunter LTD, Ringoes, NJ 08551, USA) in 8 ml 90% (w/w) Ethanol versetzt. Die Suspension wurde durch Rakeln auf eine Polyesterfolie (Mylar, Dupont) aufgezogen. Nach Abdunsten des Lösungsmittels entstand ein pH-sensitiver Film einer Schichtdicke von ca. 20 $\mu$m.

Abb. 2 zeigt die pH Abhängigkeit des Phasensignals des so hergestellten optischen Sensors.

| | | | | |
|---|---|---|---|---|
| pH 7.5 Puffer: | Luftgesättigt: | 49.3° | O2-fiei ($SO_3^{2-}$): | 51.3° |

Der Vergleich dieses Meßergebnisses mit den Daten aus 1.1 zeigt, daß ein optischer pH Sensor mit "geschützt" in einer erfindungsgemäßen Donorphase vorliegender Donorfarbstoff, eine geringere 02 Empfindlichkeit aufweist (Änderung des Phasenwinkels 2.0° beim Übergang 02-frei nach Luftsättigung) als der nach dem Stand der Technik in der Akzeptorphase vorliegende Donorfarbstoff.

Beispiel 3 ($CO_2$-Sensor)

Bei diesem Sensor werden die lumineszierenden Nanopartikel in einen Polymerfilm eingebaut, in welchem der indirekt CO2 sensitive Absorptionsindikator gelöst vorliegt. Der Unterschied zum pH Sensor (2,4) besteht im wesentlichen darin, daß jetzt keine kovalente Kopplung des analytsensitiven Absorptionsfarbstoffs an den Nanopartikeln erfolgt.

3.1 Einbetten eines erfindungsgemäß verwendeten Donorfarbstoffs in eine Akzeptorphase 5 g Ethylcellulose (Ethoxyl-Gehalt 46%, Aldrich, Steinheim, Germany) wurden in 100 ml Tetraoctylammoniumhydroxid (611 mmol/kg Ethylcellulose) und 1 mg m-Kresolpurpur-(24 mmol/kg Ethylcellulose) (m-Kresolpurpur = Tridodecylmethylammoniumsalz, TDMA) zugesetzt. Zu dieser Lösung wurden 0.7 mg des Donorfarbstoffs $Ru(diph\text{-}bpy)_3TMS_2$ (= Ruthenium(II)tris-(4,4'-diphenyl-2,2'-bipyridyl)(3-trimethylsilyl)-1-propansufonat) zugesetzt. Die Lösung wurde mit einer Naßdicke von 125 $\mu$m durch Rackeln auf eine 125 $\mu$m dicke Polyesterfolie (Mylar, Goodfellow, UK) aufgezogen. Nach Abdunsten des Lösungsmittels wurde eine ca. 10 $\mu$m dicke CO2 sensitive Schicht erhalten.

3.2 Herstellungsvorschrift der CO2 sensitiven Schicht eines optischen CO2 Sensors 5 g Ethylcellulose (Aldrich) wurden in Toluol/Ethanol (20/80, v/v) gelöst. Zu 1 g dieser Lösung wurden 100 μL Tetraoctylammoniumhydroxid (611 mmol/kg Etylcellulose) und 1.08 mg Cresolpurple-TDMA (24 mmol/kg Ethylcellulose) zugesetzt. Zu dieser Lösung wurden 700 μL (enthaltend 10.1 mg Trockengewicht an Partikel) der ethanolischen Partikel Suspension (Herstellung analog 1.2, jedoch mit dem Farbstoff $Ru(diph\text{-}bpy)_3TMS_2$) zugemischt. Die Suspension wurde mit einer Naßdicke von 125 μm durch Rackeln auf eine 125 μm dicke Polyesterfolie (Mylar, Goodfellow, UK) aufgezogen. Nach Abdunsten des Lösungsmittels wurde eine ein ca. 10 μm dicke CO2 sensitive Schicht erhalten.

Die Meßergebnisse sind in den Abbildungen 3 und 4 bzw. Tabelle 4 gezeigt. Es ist den Ergebnissen deutlich zu sehen, daß ein auf dem FRET Prinzip beruhender erfindungsgemäßer CO2 Sensor eine signifikant geringere 02-Empfindlichkeit aufweist als ein nach dem Stand der Technik hergestellter Sensor.

Tabelle 4

Vergleich der beiden Sensoren aus Abb. 1 (C1) und Abb. 2 (C2). Aus den Fehlerangaben (beide rechte Spalten) geht deutlich hervor, daß der 02 Fehler beim erfindungsgemäßen Sensor signifikant kleiner ist.

| | C1 Abklingzeit (μs) | | C2 Abklingzeit (μs) | | Angegebener $pCO_2$ Fehler in hPa | |
|---|---|---|---|---|---|---|
| $pCO_2$ (hPa) | sauerstoffrei | luftgesättigt | sauerstofffrei | luftgesättigt | C1 | C2 |
| 0 | 1.05 | 0.74 | 2.19 | 2.14 | + 7 | + 1 |
| 30 | 2.17 | 1.44 | 3.29 | 3.12 | > 800 | + 12 |
| 60 | 2.41 | 1.56 | 3.52 | 3.36 | > 800 | + 15 |
| 100 | 2.71 | 1.70 | 3.81 | 3.61 | > 800 | + 46 |

Beispiel 4 (CO2-Sensor)

Dieser Sensor besteht aus einer nanoporösen Membran (Teflonmembran von Millipore), die mit einem dünnen Film an Polyacrylnitril, in dem der Donorfarbstoff eingebaut ist, überzogen wird. Dies erfolgt durch einfaches Tränken der porösen Membran mit dem Donorcocktail und anschließenden Verdampfen des Lösungsmittels. Anschließend wird ein zweiter Überzug mit der analytsensitiven Farbchemie (in diesem Fall ein $CO_2$-Sensor) hergestellt. Damit liegen Donor und Akzeptor in getrennten Phasen vor.

Herstellungsvorschrift:

Ein nanoporöser Teflonfilter wird mit einer 5%igen Polyacrylnitril-Rutheniumkomplexlösung getränkt. Anschließend läßt man das Lösungsmittel verdunsten. (100°C für 10 Stunden). In einem 2. Schritt wird die gleiche poröse Membran mit dem CO2 sensitiven Cocktail getränkt. Der Cocktail hat folgende Zusammensetzung:

| | |
|---|---|
| Ethylcellulose: | 1 g |
| Ethanol: | 4 ml |
| Toluen: | 16 ml |
| Tetraoctylammoniumhydroxid | 250 mg |
| m-Kresolpupur-TDMA | 30 mg |

Danach wird das Filter wieder getrocknet, und der Sensor ist einsatzbereit. 30 % Abnahme der Abklingzeit zwischen 100 % CO2 und 0 % CO2.

## Patentansprüche

1. Optisch-chemischer Sensor, der nach dem FRET-Prinzip arbeitet und einen auf einen in einem Probenmedium enthaltenen Analyt reagierenden Akzeptor (Chromophor oder Luminophor) sowie einen Donor (Luminophor) aufweist, **dadurch gekennzeichnet, dass** der Akzeptor und der Donor jeweils in einem Matrixmaterial vorliegen und sich in voneinander getrennten chemischen Phasen befinden, wobei die den Donor enthaltende Phase für das Probenmedium oder für die Lumineszenzeigenschaften des Luminophors beeinflussende Komponenten des Probenmediums im wesentlichen impermeabel ist.

2. Sensor gemäß Anspruch 1, **dadurch gekennzeichnet, dass** das Matrixmaterial der Akzeptor- und Donorphasen gemischt in bzw. in Form einer dünnen Schicht (Sensorschicht) vorliegt.

3. Sensor gemäß Anspruch 2, **dadurch gekennzeichnet, daß** die Sensorschicht auf einem transparenten Substrat oder an einem Lichtleiter befestigt ist.

4. Sensor gemäß Anspruch 2 oder 3, **dadurch gekennzeichnet, daß** eine für den Analyten permeable optische Isolierschicht vorgesehen ist, welche sich zwischen der Sensorschicht und der zu analysierenden Substanz befindet.

5. Sensor gemäß einem der Ansprüche 2 bis 4, **dadurch gekennzeichnet, daß** die Sensorschicht aus einem dünnen porösen Material besteht, wobei die Poren dieses Materials das Matrixmaterial für die Donorphase und das Matrixmaterial für die Akzeptorphase enthalten.

6. Sensor gemäß einem der Ansprüche 2 bis 4, **dadurch gekennzeichnet, daß** die Sensorschicht aus einem dünnen Film gebildet wird, wobei der dünne Film das Matrixmaterial der Akzeptorphase darstellt.

7. Sensor gemäß Anspruch 6, **dadurch gekennzeichnet, daß** das Matrixmaterial der Donorphase im Matrixmaterial der Akzeptorphase homogen verteilt vorliegt.

8. Sensor gemäß einem der Ansprüche 2 bis 7, **dadurch gekennzeichnet, daß** die Donorphase in Form von Partikeln vorliegt.

9. Sensor gemäß Anspruch 8, **dadurch gekennzeichnet, daß** die Akzeptormoleküle an die Oberfläche der Partikeln der Donorphase gebunden sind.

10. Sensor gemäß einem der vorhergehenden Ansprüche, **dadurch gekennzeichnet, daß** als Material für die Donorphase nicht weichgemachte Polymere eingesetzt werden.

11. Sensor gemäß Anspruch 10, **dadurch gekennzeichnet, daß** als Polymer Polyacrylnitril und Derivate davon, PVC und/oder Polyvinylidenchlorid eingesetzt werden.

12. Sensor gemäß einem der vorhergehenden Ansprüche, **dadurch gekennzeichnet, daß** als Material für die Akzeptorphase Polyvinylchlorid, Polystyrole, Polycarbonate, Polyethylene, Polyurethane, Silikone, Copolymere aus Polyvinylakohol und Polyvinylacetat, Copolymere aus Polyvinylchlorid, Polyvinylalkohol und Polyvinylacetat, Zellulose, Polyurethane mit hydrophilen Gruppen, Polyhydroxyethylmethacrylate, vernetzte Polyvinylalkohole, und/oder Polyacrylamide, gegebenenfalls mit bis zu 80% Weichmacher, eingesetzt werden.

13. Verfahren zur qualitativen und/oder quantitativen Bestimmung zumindest eines Analyten bzw. einer Komponente eines gasförmigen oder flüssigen Meßmediums nach dem FRET-Prinzip, **gekennzeichnet durch** die Verwendung eines Sensors gemäß einem der Ansprüche 1 bis 12.

14. Verfahren gemäß Anspruch 13, **dadurch gekennzeichnet, daß** es zur Bestimmung des pH-Wertes einer Probe, zur Bestimmung der Konzentration bzw. der Aktivität von Ionen in einer Probe oder zur Bestimmung von in wässerigen Medien sauer oder alkalisch reagierenden, unter Normalbedingungen gasförmig vorliegenden Komponenten durchgeführt wird.

15. Verfahren gemäß Anspruch 14, **dadurch gekennzeichnet, daß** es zur Bestimmung der Konzentration bzw. Aktivität von $Li^+$, $Na^+$, $K^+$, $Mg^{++}$, $Ca^{++}$ oder $Cl^-$ durchgeführt wird.

16. Verfahren gemäß Anspruch 14, **dadurch gekennzeichnet, daß** es zur Bestimmung von $CO_2$ oder $NH_3$ in einem flüssigen Meßmedium durchgeführt wird.

17. Verfahren gemäß einem der Ansprüche 13 bis 16, **dadurch gekennzeichnet, daß** das Meßmedium eine Körperflüssigkeit, insbesondere Blut, Plasma oder Serum, ist.

18. Verfahren gemäß einem der Ansprüche 13 bis 17, **dadurch gekennzeichnet; daß** der Sensor als Transducer

eingesetzt wird.

**19.** Verfahren gemäß Anspruch 18, **dadurch gekennzeichnet, daß** ein enzymatischer Sensor eingesetzt wird.

## Claims

**1.** An optochemical sensor functioning in accordance with the FRET-principle and exhibiting an acceptor (chromophore or luminophore) responsive to an analyte contained in a sample medium as well as a donor (luminophore), **characterized in that** the acceptor and the donor are each present in a matrix material and are located in separate chemical phases, whereby the phase containing the donor is essentially impermeable to the sample medium or to components of the sample medium affecting the luminescence characteristics of the luminophore.

**2.** A sensor according to claim 1, **characterized in that** the matrix material of the acceptor and donor phases is provided in mixed form or in the form of a thin layer (sensor layer), respectively.

**3.** A sensor according to claim 2, **characterized in that** the sensor layer is attached to a transparent substrate or to a light guide.

**4.** A sensor according to claim 2 or 3, **characterized in that** an optical insulation layer permeable to the analyte and located between the sensor layer and the substance to be analyzed is provided.

**5.** A sensor according to any of claims 2 to 4, **characterized in that** the sensor layer consists of a thin porous material, the pores of that material containing the matrix material of the donor phase and the matrix material of the acceptor phase.

**6.** A sensor according to any of claims 2 to 4, **characterized in that** the sensor layer is composed of a thin film, said thin film representing the matrix material of the acceptor phase.

**7.** A sensor according to claim 6, **characterized in that** the matrix material of the donor phase is provided in homogeneously distributed fashion in the matrix material of the acceptor phase.

**8.** A sensor according to any of claims 2 to 7, **characterized in that** the donor phase is provided in the form of particles.

**9.** A sensor according to claim 8, **characterized in that** the acceptor molecules are attached to the surface of the particles of the donor phase.

**10.** A sensor according to any of the preceding claims, **characterized in that** unplasticized polymers are used as a material for the donor phase.

**11.** A sensor according to claim 10, **characterized in that** polyacryl nitrile and derivatives thereof, PVC and/or polyvinylidene chloride are used as polymers.

**12.** A sensor according to any of the preceding claims, **characterized in that** polyvinyl chloride, polystyrenes, polycarbonates, polyethylenes, polyurethanes, silicones, copolymers of polyvinyl alcohol and polyvinyl acetate, copolymers of polyvinyl chloride, polyvinyl alcohol and polyvinyl acetate, cellulose, polyurethanes with hydrophilic groups, polyhydroxyethylmethacrylates, crosslinked polyvinyl alcohols, and/or polyacrylamides, optionally with up to 80% of plasticizer, are used as materials for the acceptor phase.

**13.** A method for qualitative and/or quantitative determination of at least one analyte and/or component of a gaseous or liquid measuring medium according to the FRET-principle, **characterized by** the use of a sensor according to any of claims 1 to 12.

**14.** A method according to claim 13, **characterized in that** it is carried out for the determination of the pH-value of a sample, for the determination of concentrations and/or activities of ions in a sample or for the determination of components exhibiting acid or alkaline reactions in aqueous media while being gaseous under normal conditions.

**15.** A method according to claim 14, **characterized in that** it is carried out for the determination of concentrations and/or

activities of $Li^+$, $Na^+$, $K^+$, $Mg^{++}$, $Ca^{++}$ or $Cl^-$.

**16.** A method according to claim 14, **characterized in that** it is carried out for the determination of $CO_2$ or $NH_3$ in a liquid measuring medium.

**17.** A method according to any of claims 13 to 16, **characterized in that** the measuring medium is a body fluid, in particular blood, plasma or serum.

**18.** A method according to any of claims 13 to 17, **characterized in that** the sensor is used as a transducer.

**19.** A method according to claim 18, **characterized in that** an enzymatical sensor is used.

## Revendications

**1.** Capteur optique-chimique qui travaille selon le principe FRET et qui présente un accepteur (chromophore ou luminophore) qui réagit avec un analyte contenu dans un fluide d'échantillon, ainsi qu'un donneur (luminophore), **caractérisé en ce que**
l'accepteur et le donneur sont tous deux présents dans un matériau de matrice et sont situés dans des phases chimiques séparées l'une de l'autre, la phase contenant le donneur étant essentiellement imperméable au fluide d'échantillon ou aux composants du fluide d'échantillon qui influent sur les propriétés de luminescence de luminophore.

**2.** Capteur selon la revendication 1, **caractérisé en ce que** le matériau de matrice des phases d'accepteur et de donneur est mélangé dans une mince couche (couche de capteur) ou présente la forme d'une mince couche.

**3.** Capteur selon la revendication 2, **caractérisé en ce que** la couche de capteur est fixée sur un substrat transparent ou sur un conducteur de lumière.

**4.** Capteur selon les revendications 2 ou 3, **caractérisé en ce qu'**il présente une couche d'isolation optique perméable aux analytes et située entre la couche de capteur et la substance à analyser.

**5.** Capteur selon l'une des revendications 2 à 4, **caractérisé en ce que** la couche de capteur est constituée d'un matériau à porosité fine, les pores de ce matériau contenant le matériau de matrice pour la phase de donneur et le matériau de matrice pour la phase d'accepteur.

**6.** Capteur selon l'une des revendications 2 à 4, **caractérisé en ce que** la couche de capteur est formée d'un film mince, le film mince constituant le matériau de matrice de la phase d'accepteur.

**7.** Capteur selon la revendication 6, **caractérisé en ce que** le matériau de matrice de la phase de donneur est réparti de manière homogène dans le matériau de matrice de la phase d'accepteur.

**8.** Capteur selon l'une des revendications 2 à 7, **caractérisé en ce que** la phase de donneur présente la forme de particules.

**9.** Capteur selon la revendication 8, **caractérisé en ce que** les molécules d'accepteur sont liées à la surface des particules de la phase de donneur.

**10.** Capteur selon l'une des revendications précédentes, **caractérisé en ce qu'**il utilise des polymères non plastifiés pour le matériau de la phase de donneur.

**11.** Capteur selon la revendication 10, **caractérisé en ce qu'**il utilise comme polymère le polyacrylonitrile et ses dérivés, le PVC et/ou le poly(chlorure de vinylidène).

**12.** Capteur selon l'une des revendications précédentes, **caractérisé en ce que** comme matériau pour la phase d'accepteur, il utilise le poly(chlorure de vinyle), les polystyrènes, les polycarbonates, le polyéthylène, les polyuréthanes, les silicones, les copolymères d'alcool vinylique et d'acétate de vinyle, les copolymères de chlorure de vinyle, d'alcool vinylique et d'acétate de vinyle, la cellulose, les polyuréthanes à groupes hydrophiles, les poly(méthacrylate d'hydroxyéthyle), les poly(alcool vinylique) réticulés et/ou les polyacrylamides, éventuellement avec jusqu'à 80 % de plastifiant.

**13.** Procédé de détermination qualitative et/ou quantitative d'au moins un analyte d'un composant d'un fluide gazeux ou liquide à mesurer selon le principe FRET, **caractérisé par** le recours à un capteur selon l'une des revendications 1 à 12.

**14.** Procédé selon la revendication 13, **caractérisé en ce qu'**il est exécuté pour déterminer la valeur du pH d'un échantillon, pour déterminer la concentration ou l'activité d'ions dans un échantillon ou pour déterminer des composants présents sous forme gazeuse dans des conditions normales et réagissant de façon acide ou basique dans des milieux aqueux.

**15.** Procédé selon la revendication 14, **caractérisé en ce qu'**il est exécuté pour déterminer la concentration ou l'activité de $Li^+$, $Na^+$, $K^+$, $Mg^{++}$, $Ca^{++}$ ou $Cl^-$.

**16.** Procédé selon la revendication 14, **caractérisé en ce qu'**il est exécuté pour déterminer la présence de $CO_2$ ou de $NH_3$ dans un milieu liquide à mesurer.

**17.** Procédé selon l'une des revendications 13 à 16, **caractérisé en ce que** le milieu à mesurer est un liquide corporel, en particulier le sang, le plasma ou le sérum.

**18.** Procédé selon l'une des revendications 13 à 17, **caractérisé en ce que** le capteur est utilisé comme transducteur.

**19.** Procédé selon la revendication 18, **caractérisé en ce qu'**il utilise un capteur enzymatique.

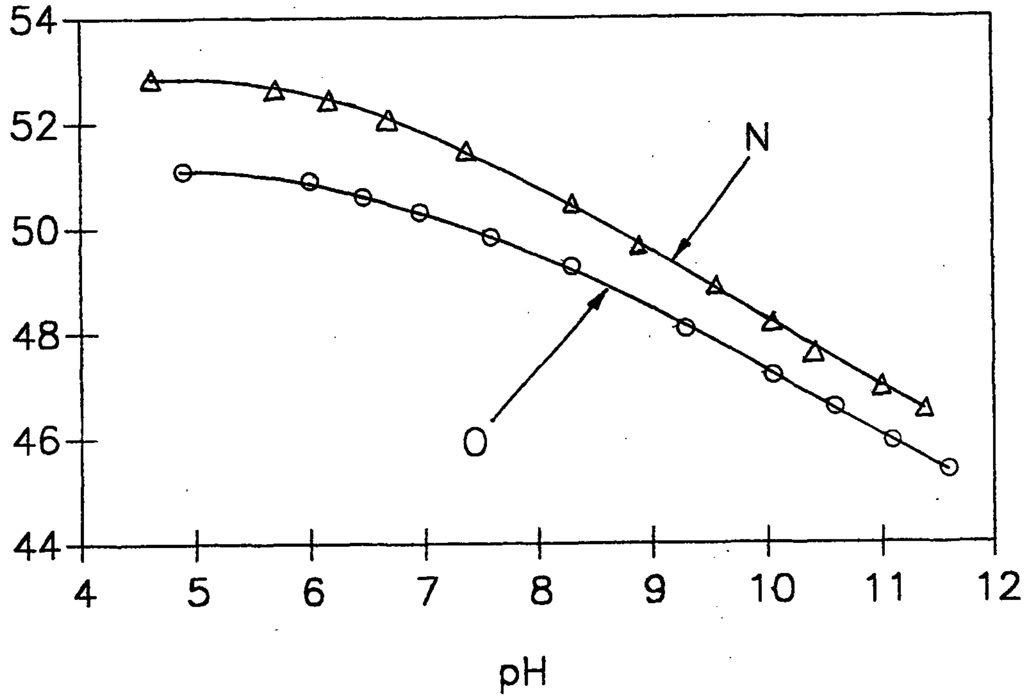


FIG. 1

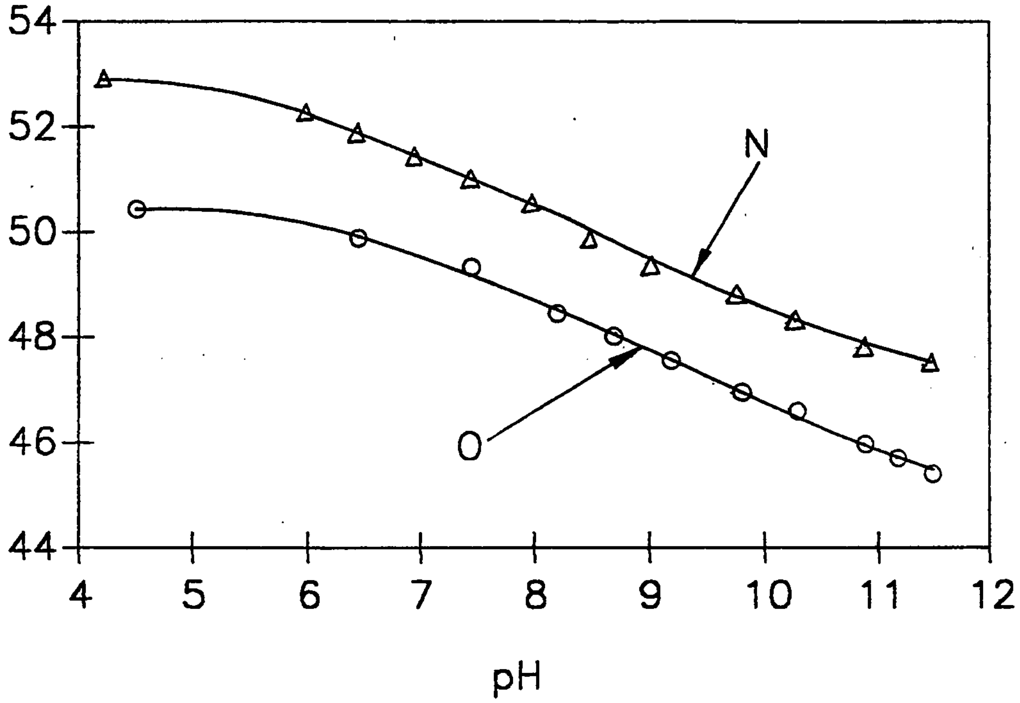


FIG. 2

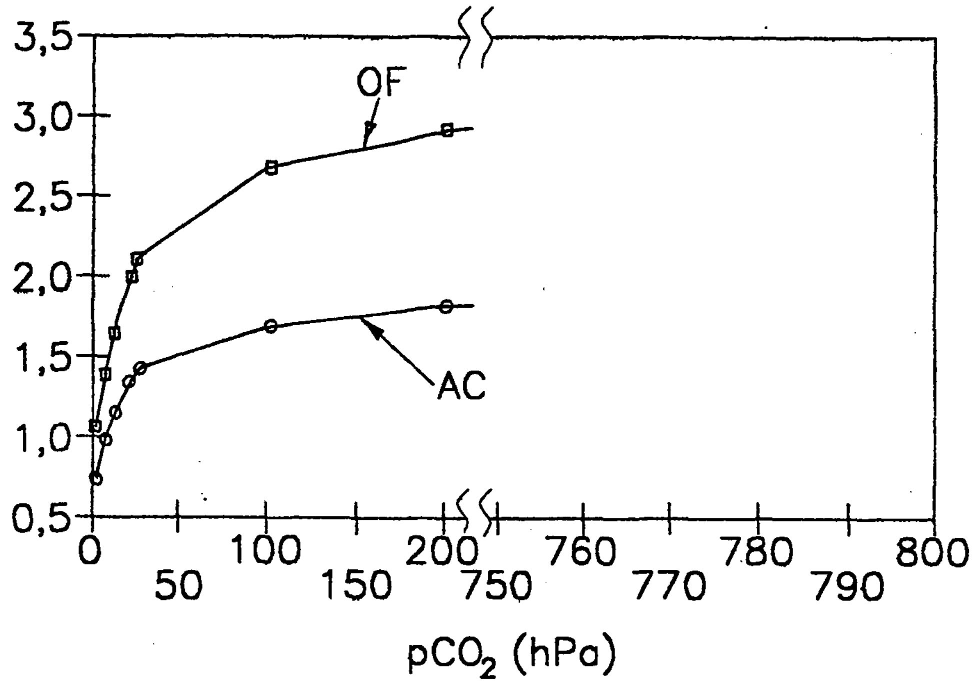
3,5
3,0
2,5
2,0
1,5
1,0
0,5
OF
AC
0
50
100
150
200
750
760
770
780
790
800
pCO2 (hPa)

FIG. 3

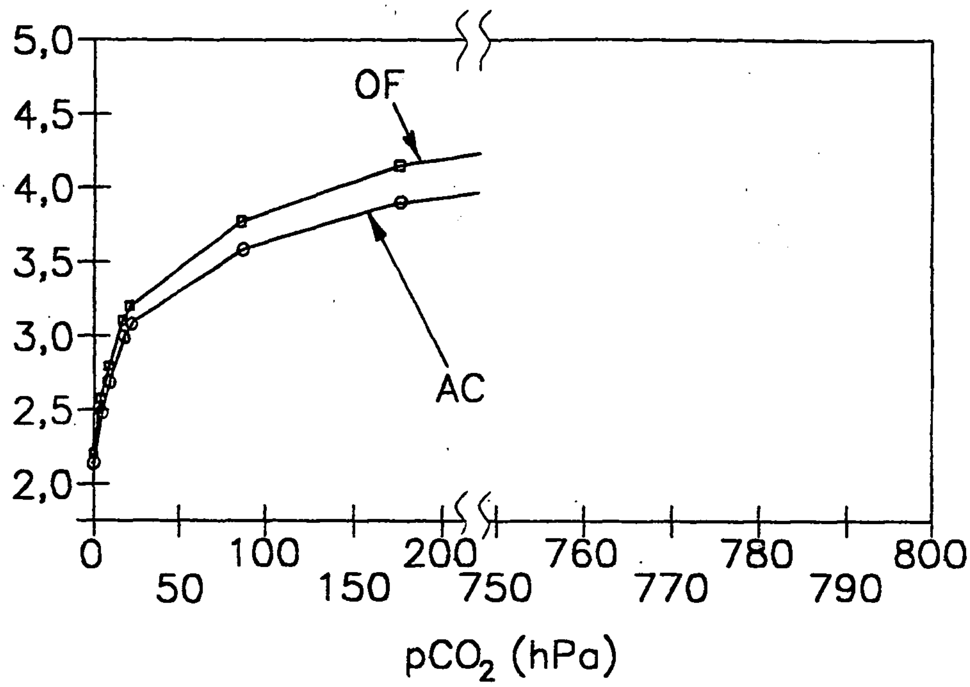
5,0
4,5
4,0
3,5
3,0
2,5
2,0
OF
AC
0
50
100
150
200
750
760
770
780
790
800
pCO2 (hPa)

FIG. 4

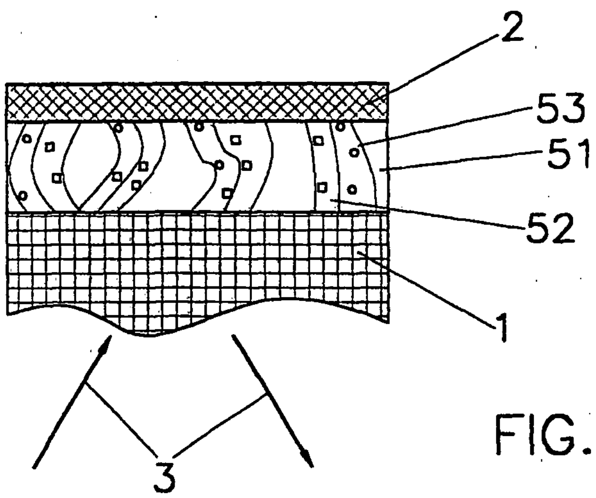


FIG. 5

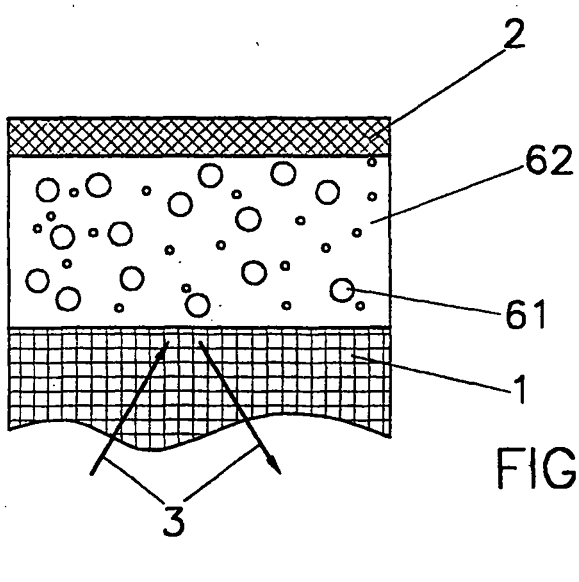


FIG. 6

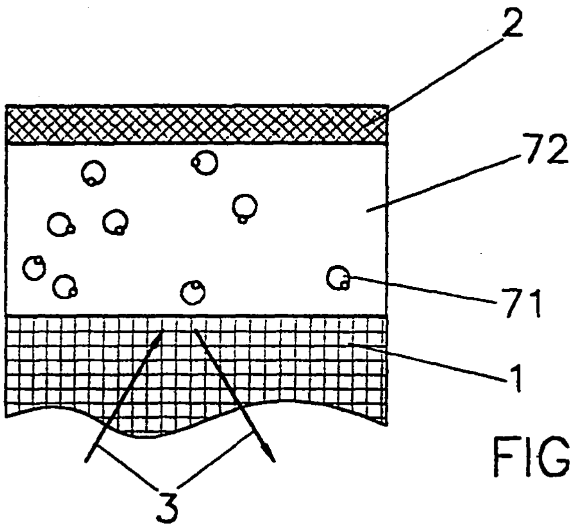


FIG. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5232858 A
- US 5648269 A
- EP 0214768 A
- US 4868103 A
- US 5194393 A
- US 5711915 A
- US 5942189 A
- US 5114676 A
- US 4557900 A
- WO 02054476 A
- US 6046055 A
- EP 0907074 A
- US 4645744 A
- EP 0358991 A
- EP 0461392 A
- EP 0105870 A
- US 5496521 A

### In der Beschreibung aufgeführte Nicht-Patentliteratur

- Fiber Optic pH Sensors. **M.J.P. Leiner ; O.S. Wolfbeis.** Fiber Optic Chemical Sensors and Biosensors. CRC-Press, 1991, vol. I
- *Anal. Chim. Acta,* 1991, vol. 255, 35-44
- **Mills et al.** *Anal. Chem.,* 1992, vol. 64, 1383-1389
- **T.Werner et al.** *Analyst,* 1995, vol. 120, 1627-1631
- **K. Wanabe et al.** *Anal. Chem.,* 1993, vol. 65